# EUROPÄISCHE PATENTANMELDUNG

(11) **EP 2 189 793 A1**
(43) Veröffentlichungstag der Anmeldung: **26.05.2010**
(21) Anmeldenummer: 08020280.7
(22) Anmeldetag: 21.11.2008
(51) Int. Cl.: G01N 33/543, C12Q 1/68

(54) **Verfahren zum Regenerieren eines Biosensors**

(71) Anmelder: Micronas GmbH, 79108 Freiburg i. Br. (DE)
(72) Erfinder: Freund, Ingo, Dipl.-Ing., 79117 Freiburg (DE); Bednar, Sonja, 79194 Gundelfingen (DE); Klapproth, Holger, Dr., 79108 Freiburg (DE); Baader, Johannes, Dipl.-Ing., 79098 Freiburg (DE)
(74) Vertreter: Huwer, Andreas

(57) **Zusammenfassung**

Bei einem Verfahren zum Regenerieren eines Biosensors (1) wird ein Biosensor (1) bereitgestellt, der eine Trägeroberfläche (3) aufweist, auf der wenigstens ein Rezeptor (5a, 5b) immobilisiert ist. An den Rezeptor (5a, 5b) ist mindestens ein für den Rezeptor (5a, 5b) bindungsspezifischer Ligand (7a, 7b) gebunden, der mit dem Rezeptor (5a, 5b) einen Ligand-Rezeptor-Komplex bildet. Zum Regenerieren des Biosensors (1) wird der Ligand-Rezeptor-Komplex mit einem Enzym (8) in Kontakt gebracht. Das Enzym (8) wird derart gewählt, dass es den Ligand (7a, 7b) in Fragmente (9) katalysiert. Gegenüber dem Rezeptor (5a, 5b) ist das Enzym (8) inert.

## Beschreibung

Die Erfindung betrifft ein Verfahren zum Regenerieren eines Biosensors, wobei ein Biosensor bereitgestellt wird, der eine Trägeroberfläche aufweist, auf der wenigstens ein Rezeptor immobilisiert ist, an den mindestens ein für den Rezeptor bindungsspezifischer Ligand gebunden ist, der mit dem Rezeptor einen Ligand-Rezeptor-Komplex bildet, und wobei der Biosensor regeneriert wird, indem der Ligand vom Rezeptor getrennt wird.

Ein derartiges Verfahren ist aus EP 1 078 248 B1 bekannt. Dabei wird zunächst ein Biosensor bereitgestellt, der eine Trägeroberfläche aufweist, auf der als Rezeptoren 22-mer Oligonukleotide immobilisiert sind. Die Rezeptoren sind kovalent an reaktive Gruppen eines an der Trägeroberfläche befindlichen Epoxysilans gebunden. Die Trägeroberfläche wird mit einer Pufferlösung gespült, die einen pH-Wert von 7,75 aufweist. Danach wird eine zu untersuchende Probe auf die Trägeroberfläche aufgebracht, die einen nachzuweisenden Liganden enthält, dessen Konzentration bekannt ist. Bei dem Liganden handelt es sich um ein komplementäres 22-mer Oligonukleotid, das mit einem optischen Marker markiert ist. Die Zeitdauer, während der der Ligand mit der Trägeroberfläche in Kontakt steht, wird so groß gewählt, dass der Ligand an den Rezeptor binden kann. Dann wird eine von der Bindung des Liganden an den Rezeptor abhängige Fluoreszenzstrahlung erzeugt und mit Hilfe eines optischen Sensors gemessen. Der so erhaltene Messwert wird zwischengespeichert.

Nun wird der Träger mit der Pufferlösung gespült und es wird die Dissoziationsrate der auf der Oberfläche befindlichen DNA-Hybride ermittelt. In einem weiteren Verfahrensschritt wird die Trägeroberfläche mit 10mM einer Natriumhydroxyd-Lösung in Kontakt gebracht, um die an die Rezeptoren gebundenen Liganden von den Rezeptoren zu trennen und dadurch den Biosensor zu regenerieren. Der Biosensor wird nun erneut in der Pufferlösung kalibriert und es wird überprüft, ob die Rezeptoren vollständig regeneriert wurden.

Das Verfahren hat den Nachteil, dass die Liganden durch das Inkontaktbringen mit der Natriumhydroxyd-Lösung geschädigt werden können. Insbesondere kann während des Regenerierungsschritts die Bindungsaffinität der Rezeptoren für die Liganden abnehmen. Auch kann es vorkommen, dass durch das Anwenden der Natriumhydroxyd-Lösung nicht alle Liganden von den Rezeptoren abgelöst werden können. Ferner können auch der optische Sensor und/oder elektrische Bauteile, die mit dem Natriumhydroxyd-Lösung in Kontakt geraten, geschädigt werden.

Bei einem anderen, aus der Praxis bekannten Verfahren der eingangs genannten Art wird der Biosensor durch Erhitzen regeneriert. Dabei wird die Temperatur derart erhöht, dass sich die an die Rezeptoren gebunden Liganden von den Rezeptoren ablösen. Auch bei diesem Verfahren können die Rezeptoren geschädigt werden. Das Verfahren wird hauptsächlich bei DNA-Rezeptoren angewendet. Für Protein-Rezeptoren ist das Verfahren nur sehr bedingt geeignet, da Protein-Rezeptoren bei Temperaturerhöhung schnell denaturieren und dann für die Liganden nicht mehr bindungsspezifisch sind.

Es besteht deshalb die Aufgabe, ein Verfahren der eingangs genannten Art zu schaffen, das es ermöglicht, den Biosensor nach dem Binden des Liganden an den Rezeptor zu regenerieren. Dabei soll die Sensitivität des Biosensors weitestgehend erhalten bleiben.

Diese Aufgabe wird dadurch gelöst, dass der Ligand-Rezeptor-Komplex zum Regenerieren mit einem Enzym in Kontakt gebracht wird, und dass das Enzym derart gewählt wird, dass es den Liganden in Fragmente katalysiert und gegenüber dem Rezeptor inert ist.

Überraschender Weise hat sich herausgestellt, dass es mit Hilfe eines solchen Enzyms auf einfache Weise möglich ist, die Liganden nahezu vollständig von den daran gebundenen Rezeptoren zu trennen, ohne dass dabei die Rezeptoren geschädigt werden. Durch das Enzym kann sogar die an den Rezeptor gebundene Gruppe des Liganden vom Rezeptor getrennt werden. Danach können das Enzym und/oder die Fragmente des Liganden auf einfache Weise von der Trägeroberfläche entfernt werden, beispielsweise indem eine Spülflüssigkeit auf die Trägeroberfläche aufgebracht wird, um die Fragmente und das Enzym wegzuspülen. Mit Hilfe des Verfahren ist es möglich, einen Biosensor beispielsweise nach Durchrührung eines Assays zu reinigen und wiederzuverwenden, um einen Liganden in zu untersuchenden Proben nachzuweisen und/oder die Konzentration von Liganden zu messen. Dabei kann die Reinigung bzw. Verwendung des Biochips ggf. auch mehrfach erfolgen. Das erfindungsgemäße Verfahren kann auch dazu verwendet werden, Biochips nach einer Lagerung und/oder einem Transport (z.B. einer Verschiffung) vor der erstmaligen Verwendung von einer Kontamination z.B. mit Proteinen zu reinigen.

Vorteilhaft ist, wenn der Ligand und der Rezeptor unterschiedlichen Substanzklassen angehören. Wegen des dadurch bedingten unterschiedlichen Aufbaus von Rezeptor und Ligand ist der Rezeptor gegenüber einem Enzym, bei dessen Anwesenheit sich der Ligand zersetzt, besonders unempfindlich. Somit kann der Biosensor noch schonender regeneriert werden.

Bei einer bevorzugten Ausgestaltung der Erfindung ist der Rezeptor eine Ribonukleinsäure, ein Protein und/oder eine Peptidnukleinsäure und der Ligand eine Desoxyribonukleinsäure, wobei das Enzym eine Desoxyribonuklase ist. Unter einer Desoxyribonuklase wird ein Enzym verstanden, das Desoxyribonukleinsäurestränge abbaut, beispielsweise eine Exonuklease und/oder eine Endonuklease.

Bei einer anderen vorteilhaften Ausführungsform der Erfindung ist der Rezeptor ein Protein, eine Desoxyribonukleinsäure und/oder eine Peptidnukleinsäure und der Ligand eine Ribonukleinsäure, wobei das Enzym eine Ribonuklease ist. Unter einer Ribonuklase wird ein Enzym verstanden, das Ribonukleinsäurestränge abbaut, beispielsweise Ribonuklase A, welche freie Ribonukleinsäurestränge abbaut, und/oder Ribonuklase B, die an Desoxyribonukleinsäure gebundene Ribonukleinsäurestränge abbaut.

Bei einer anderen vorteilhaften Ausgestaltung der Erfindung ist der Rezeptor ein Biotin und der Ligand Streptavidin, wobei das Enzym eine Proteinase ist. Unter einer Proteinase wird ein Enzym verstanden, das Eiweiße oder Peptide spaltet bzw. in ihre Einzelteile zerlegt, beispielsweise Proteinase K und/oder Peptidase.

Es ist aber auch möglich, dass der Rezeptor ein Polysaccarid, ein Lipid, ein Digoxygenin und/oder ein Kohlehydrat und der Ligand ein Protein, insbesondere ein Antikörper ist, und dass das Enzym eine Proteinase ist. Dabei kann der Antikörper beispielsweise ein Lektin sein.

Bei einer bevorzugten Ausgestaltung der Erfindung
- wird mindestens ein Biosensor bereitgestellt, der eine Trägeroberfläche aufweist, auf der wenigstens ein für den Liganden bindungsspezifischer Rezeptor immobilisiert ist,
- wobei eine erste Probe, die den Ligand in einer bekannten ersten Konzentration enthält, derart mit dem Rezeptor in Kontakt gebracht wird, dass der mindestens eine Ligand an den Rezeptor binden und mit diesem einen Ligand-Rezeptor-Komplex bilden kann,
- wobei mindestens ein von der Bindung des Liganden an den Rezeptor abhängiger erster Messwert erfasst wird,
- wobei der Biosensor danach regeneriert wird,
- wobei dann eine zweite Probe, die den Ligand in einer zu bestimmenden zweiten Konzentration enthält, derart mit dem Rezeptor in Kontakt gebracht wird, dass der mindestens eine Ligand an den Rezeptor binden und mit diesem einen Ligand-Rezeptor-Komplex bilden kann,
- wobei mindestens ein von der Bindung des Liganden an den Rezeptor abhängiger zweiter Messwert erfasst wird,
- und dass mit Hilfe der Messwerte und der bekannten ersten Konzentration die zu bestimmende zweite Konzentration ermittelt wird.
Dadurch ist es möglich, den Biosensor exakt zu kalibrieren und somit die Konzentration der Liganden in der zweite Probe mit großer Präzision zu bestimmen.

Nachfolgend ist ein Ausführungsbeispiel der Erfindung anhand der Zeichnung näher erläutert:
- Fig. 1: eine Seitenansicht eines Biosensors, der einen Träger aufweist auf dessen Oberfläche Rezeptoren immobilisiert sind,
- Fig. 2 und 3: graphische Darstellungen von Messwerten, die jeweils von der Bindung von Liganden an Rezeptoren einer bestimmten Rezeptorart abhängig sind, wobei auf der Abszisse die Rezeptorart und auf der Ordinate der Messwert aufgetragen sind,
- Fig. 4: einen Biosensor, an dessen Rezeptoren Liganden gebunden sind,
- Fig. 5: den in Fig. 4 gezeigten Biosensor nach dem Inkontaktbringen mit einem Enzym, in dessen Anwesenheit die Liganden zerstückelt, in Monomere zerlegt bzw. verdaut werden, und
- Fig.6: den in Fig. 5 gezeigten Biosensor nachdem Liganden in Anwesenheit des Enzyms fragmentiert wurden.

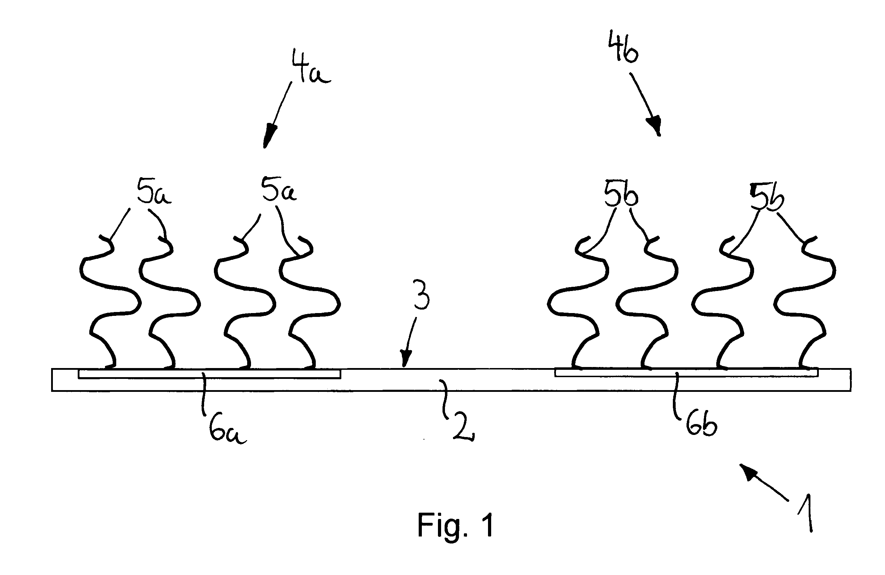

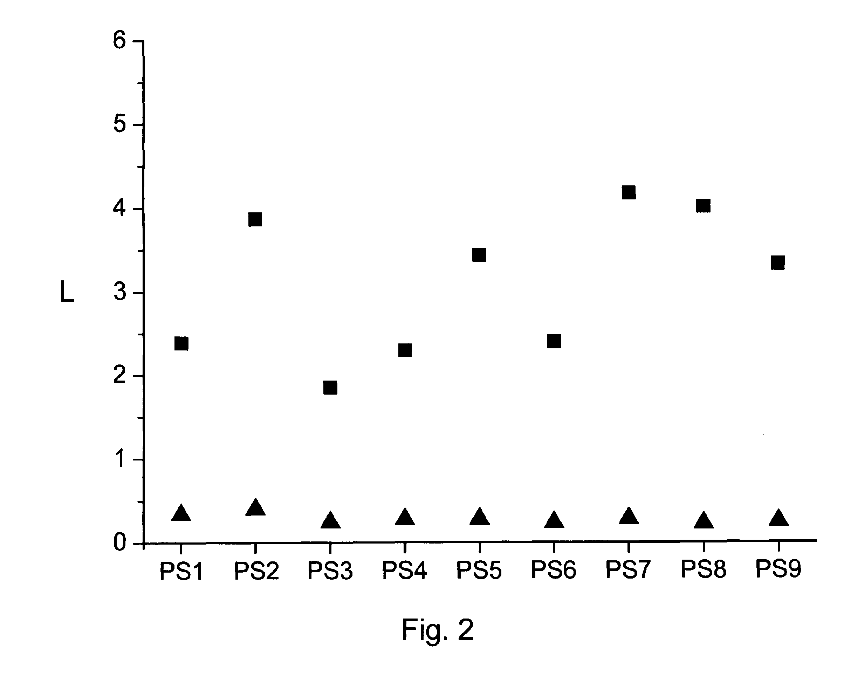

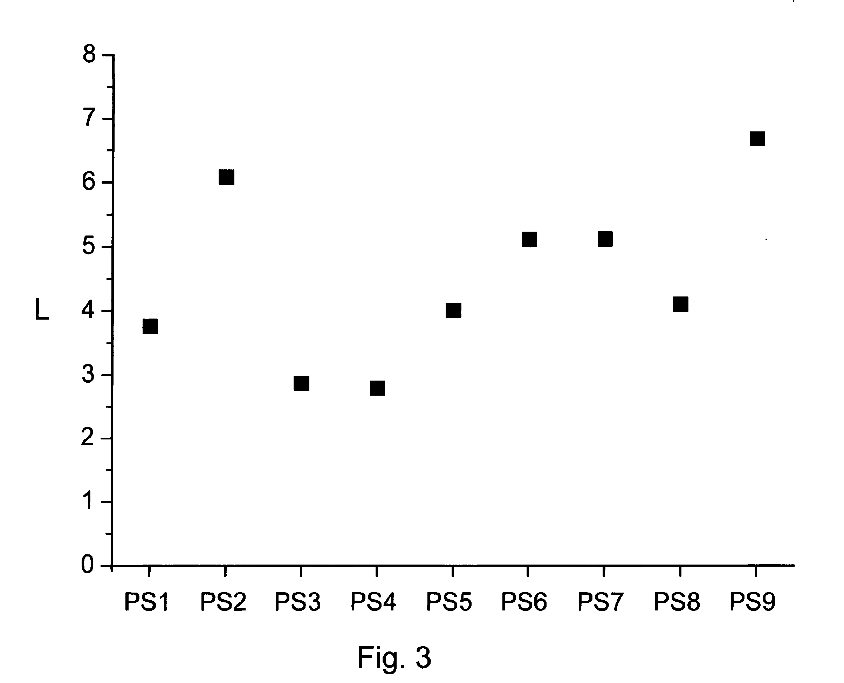

Bei einem Verfahren zum Regenerieren von Biosensoren wird ein Biosensor 1 bereitgestellt, der einen Träger 2 aufweist, auf dessen Trägeroberfläche 3 mehrere Teststellen 4a, 4b vorgesehen sind, an denen Rezeptoren 5a, 5b unterschiedlicher Rezeptorarten immobilisiert sind (Fig. 1). Bei den Rezeptoren 5a, 5b handelt es sich jeweils um Pneumokokken-Polysaccaride. Die unterschiedlichen Rezeptorarten sind in Fig. 2 und 3 mit PS1 bis PS9 bezeichnet.

Der Träger 2 besteht im Wesentlichen aus einem Halbleiterwerkstoff, vorzugsweise aus Silizium. An der Trägeroberfläche 3 weist der Träger 2 eine in der Zeichnung nicht näher dargestellte Silanschicht auf, an die erste Bindungsstellen der Rezeptoren 5a, 5b kovalent gebunden sind.

In Fig. 1 ist erkennbar, dass an den Teststellen 4a, 4b unterhalb der Rezeptoren 5a, 5b Sensoren 6 in den Träger 2 integriert sind. Die Sensoren 6 sind für eine noch näher zu erläuternde Lumineszenzstrahlung empfindlich.

Es ist auch denkbar, das der Träger 2 nur bereichsweise, insbesondere an den Stellen, an denen sich die Sensoren 6 befinden, aus dem Halbleiterwerkstoff besteht. Ferner besteht die Möglichkeit, die Sensoren 6 außerhalb des Trägers 2 anzuordnen. In diesem Fall kann der Träger 2 auch aus einem anderen für die Immobilisierung der Rezeptoren 5a, 5b geeigneten Werkstoff bestehen, beispielsweise aus Glas, Keramik oder Kunststoff

Auf die Trägeroberfläche 3 wird eine fließfähige erste Probe aufgebracht, die Liganden 7a, 7b unterschiedlicher Ligandenarten jeweils in bekannter Konzentration enthält. Jeder Ligand 7a, 7b ist jeweils für einen der auf dem Träger 2 immobilisierten Rezeptoren 5a, 5b bindungsspezifisch. Die Rezeptoren 5a, 5b weisen dazu jeweils mindesten eine zweite Bindungsstelle auf, an welche der für den betreffenden Rezeptor 5a, 5b bindungsspezifische Ligand 7a, 7b binden kann.

Die erste Probe kann beispielsweise dadurch hergestellt werden, dass bestimmte Mengen der einzelnen Liganden 7a, 7b mit einer vorbestimmten Menge eines wässrigen Lösungsmittels verdünnt werden.

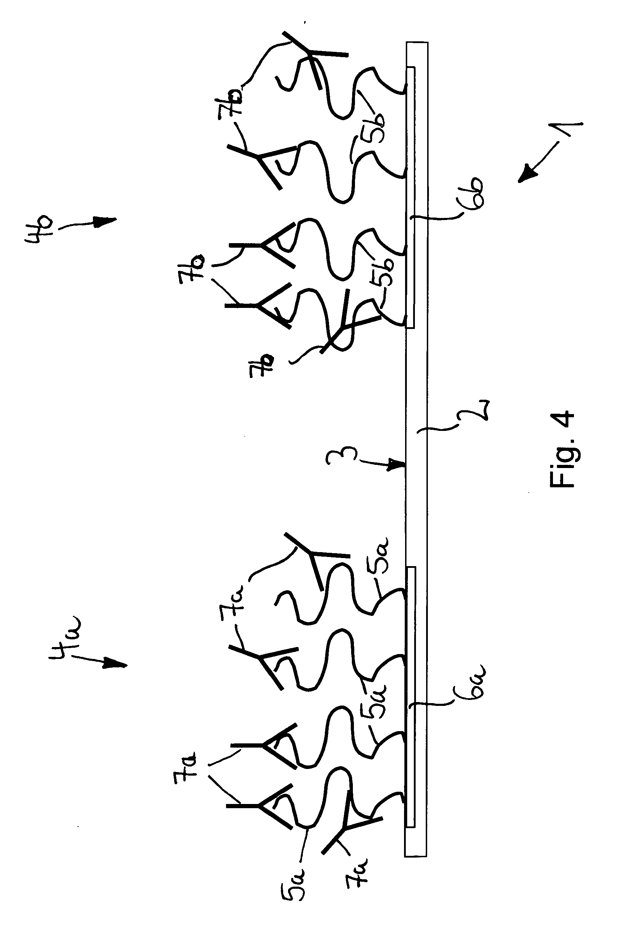

Die Zeitdauer, während der die erste Probe mit den Rezeptoren 5a, 5b in Kontakt steht, wird so gewählt, dass an nahezu alle zweiten Bindungsstellen der Rezeptoren 5a, 5b jeweils ein Ligand 7a, 7b bindet. Die so erhaltenen Rezeptor-Ligandenkomplexe sind in Fig. 4 dargestellt.

Danach werden eventuelle ungebundene Rezeptoren 5a, 5b von der Trägeroberfläche 3 entfernt, beispielsweise mit Hilfe einer Spüllösung. Dann werden an die auf der Trägeroberfläche 3 zurückgebliebenen Rezeptor-Ligandenkomplexe Nachweisantikörper gebunden, die über Streptavidin mit einem optischen Marker, nämlich Biotin markiert sind (Sandwich-ELISA). In einem weiteren Schritt werden eventuelle freie Nachweisantikörper und eventuelles ungebundenes Streptavidin von der Trägeroberfläche 3 entfernt.

Die auf der Trägeroberfläche verbleibenden Rezeptor-Liganden-Nachweisantikörper-Biotin-Komplexe werden mit einer streptavidinkonjugierten Meerrettichperoxidase-Lösung in Kontakt gebracht. Nach Zugabe eines Chemilumineszenzsubstrats wird die Meerrettichperoxidase durch eine chemische Reaktion zur Aussendung einer Lumineszenzstrahlung angeregt. Diese wird jeweils mit Hilfe des der betreffenden Teststelle 4a, 4b zugeordneten Sensors 6a, 6b gemessen. Die einzelnen Teststellen sind derart voneinander beabstandet, dass die von einer Teststelle ausgesendete Lumineszenzstrahlung für die Sensoren benachbarter Teststellen 4b, 4a nicht sichtbar ist. In Fig. 2 sind die so erhaltenen ersten Messwerte durch quadratische Punkte markiert. Deutlich ist erkennbar, dass die einzelnen Sensoren 6a, 6b jeweils ein Messsignal aufweisen.

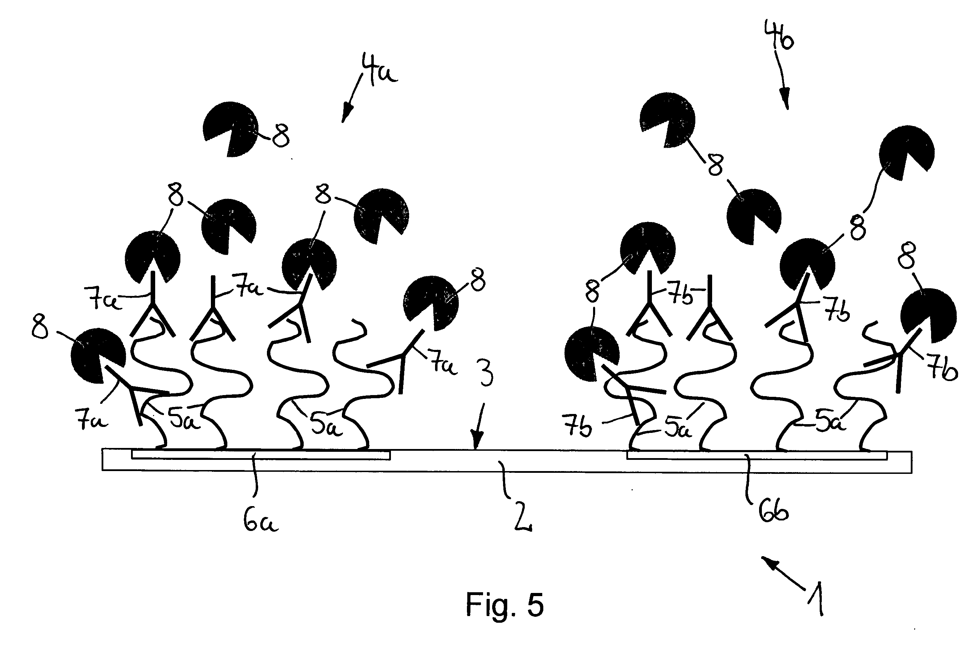

Nachdem auf diese Weise die Messwerte erfasst wurden, wird die Trägeroberfläche 3 gespült und mit einer ein Proteinase-K-Enzym 8 enthaltenden Lösung für etwa 4 Stunden bei einer Temperatur von circa 56°C in Kontakt gebracht (Fig. 5). Das Enzym 8 ist derart gewählt, dass die Liganden 7a, 7b bei Anwesenheit des Enzyms 8 in Fragmente katalysiert werden. Gegenüber den Rezeptoren 5a, 5b ist das Enzym 8 dagegen inert, d.h. die Rezeptoren 5a, 5b werden durch die Anwesenheit des Enzyms nicht verändert. Das Enzym 8 ist im Handel beispielsweise bei dem Unternehmen Sigma-Aldrich^{™}, Saint Louis, MO 63103 USA, unter der Katalog-Nummer P2308 erhältlich.

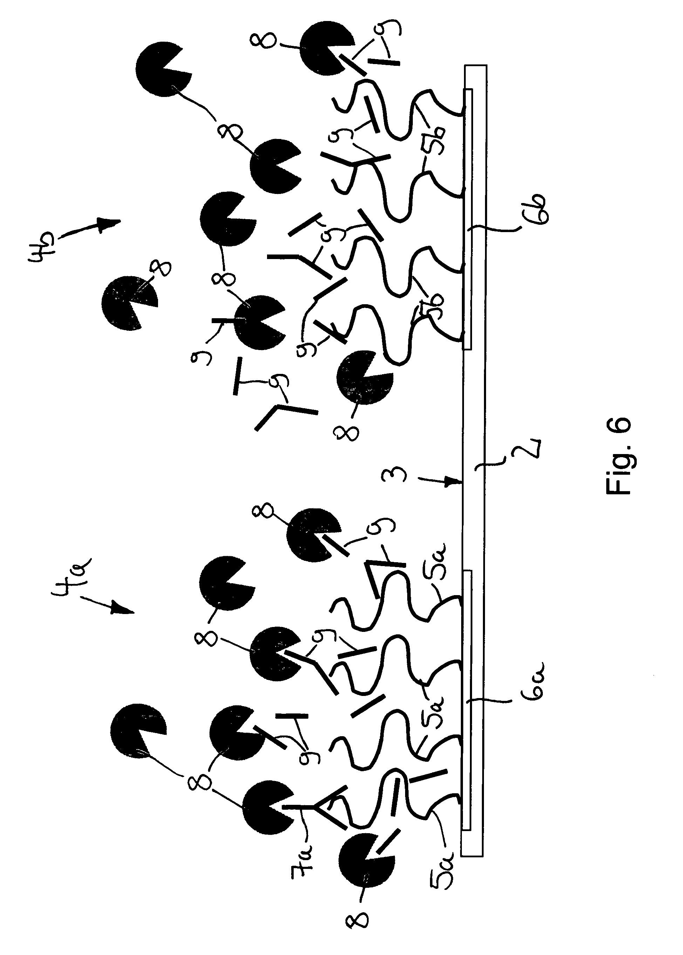

Wie in Fig. 6 erkennbar ist, werden die Liganden 7a, 7b durch die Fragmentierung vollständig von den zweiten Bindungsstellen der Rezeptoren 5a, 5b getrennt. Nachdem die Fragmentierung abgeschlossen ist, werden die Fragmente 9 und das Enzym von der Trägeroberfläche 3 abgespült, so dass dann nur noch die Rezeptoren 5a, 5b auf der Trägeroberfläche 3 zurückbleiben (Fig. 1).

Um die Wirkung dieser Reinigung zu überprüfen, wird mit dem gereinigten Biosensor 1 ein weiterer Versuch durchgerührt bei dem anstelle der ersten Probe eine Pufferlösung auf die Trägeroberfläche 3 aufgebracht wird, die keine Liganden 7a, 7b enthält. Dann wird der Biosensor 1 mit einer Lösung von Biotin-markierten Nachweisantikörpern in Kontakt gebracht. Nach einem weiteren Spülschritt wird der Biosensor 1 mit Streptavidin-konjugierter Meerrettichperoxidase-Lösung beaufschlagt. Dann wird wiederum das Chemilumineszenzsubstrat mit den immobilisierten Rezeptoren 5a, 5b in Kontakt gebracht und mit Hilfe der Sensoren 6a, 6b werden erneut Messwerte erfasst. Diese sind in Fig. 2 durch Dreiecke markiert. Deutlich ist erkennbar, dass bis auf eine Hintergrundstrahlung keine Lumineszenzstrahlung mehr auftritt. Die Liganden 7a, 7b wurden also vollständig von den Rezeptoren 5a, 5b entfernt.

Nachdem das Chemilumineszenzsubstrat von der Trägeroberfläche 3 des gereinigten Biochips entfernt wurde, wird eine zu untersuchende zweite Probe auf die Trägeroberfläche 3 aufgebracht, von der vermutet wird, dass sie die Liganden 7a, 7b enthält. Die Zeitdauer, während der die zweite Probe mit den Rezeptoren 5a, 5b in Kontakt steht, wird so gewählt, dass die Liganden 7a, 7b an die Rezeptoren 5a, 5b binden können.

Danach werden eventuelle ungebundene Rezeptoren 5a, 5b von der Trägeroberfläche 3 entfernt und an die auf der Trägeroberfläche 3 zurückgebliebenen Rezeptor-Ligandenkomplexe werden Nachweisantikörper gebunden, die mit Biotin markiert sind. In einem weiteren Schritt werden eventuelle freie Nachweisantikörper und eventuelles ungebundenes Biotin von der Trägeroberfläche 3 beispielsweise durch Spülen entfernt.

Die auf der Trägeroberfläche 3 verbleibenden Rezeptor-Liganden-Nachweisantikörper-Biotin-Komplexe werden mit einer streptavidinkonjugierten Meerrettichperoxidase-Lösung in Kontakt gebracht. Nach Zugabe eines Chemilumineszenzsubstrats wird die Meerreitichperoxidase durch eine chemische Reaktion zur Aussendung einer Lumineszenzstrahlung angeregt, für die mit Hilfe der Sensoren 6a, 6b zweite Messwerte erfasst werden.

In Fig. 3 sind die zweiten Messwerte durch quadratische Punkte markiert. Durch einen Vergleich von Fig. 2 mit Fig. 3 wird deutlich, dass die Sensitivität des Biosensors 1 beim Regenerieren des Biosensors 1 weitestgehend erhalten bleibt

Mit Hilfe der ersten Messwerte, der zweiten Messwerte und der bekannten Konzentrationen der in der ersten Probe enthaltenen Liganden 7a, 7b werden die Konzentrationen der Liganden 7a, 7b in der zweiten Probe ermittelt.

Bei Bedarf kann der Biosensor 1 mindesten einmal erneut regeneriert werden, um danach für zumindest eine weitere Probe Konzentrationsmesswerte für die Liganden 7a, 7b zu ermitteln.

## Patentansprüche

1. Verfahren zum Regenerieren eines Biosensors (1), wobei ein Biosensor (1) bereitgestellt wird, der eine Trägeroberfläche (3) aufweist, auf der wenigstens ein Rezeptor (5a, 5b) immobilisiert ist, an den mindestens ein für den Rezeptor (5a, 5b) bindungsspezifischer Ligand (7a, 7b) gebunden ist, der mit dem Rezeptor (5a, 5b) einen Ligand-Rezeptor-Komplex bildet, und wobei der Biosensor (1) regeneriert wird, indem der Ligand (7a, 7b) vom Rezeptor (5a, 5b) getrennt wird, **dadurch gekennzeichnet, dass** der Ligand-Rezeptor-Komplex zum Regenerieren mit einem Enzym (8) in Kontakt gebracht wird, und dass das Enzym (8) derart gewählt wird, dass es den Liganden (7a, 7b) in Fragmente (9) katalysiert und gegenüber dem Rezeptor (5a, 5b) inert ist.

2. Verfahren nach Anspruch 1, **dadurch gekennzeichnet, dass** der Ligand (7a, 7b) und der Rezeptor (5a, 5b) unterschiedlichen Substanzklassen angehören.

3. Verfahren nach Anspruch 1 oder 2, **dadurch gekennzeichnet, dass** der Rezeptor (5a, 5b) eine Ribonukleinsäure, ein Protein und/oder eine Peptidnukleinsäure und der Ligand (7a, 7b) eine Desoxyribonukleinsäure ist, und dass das Enzym (8) eine Desoxyribonuklase ist.

4. Verfahren nach einem der Ansprüche 1 bis 3, **dadurch gekennzeichnet, dass** der Rezeptor (5a, 5b) ein Protein, eine Desoxyribonukleinsäure und/oder eine Peptidnukleinsäure und der Ligand (7a, 7b) eine Ribonukleinsäure ist, und dass das Enzym (8) eine Ribonuklease ist.

5. Verfahren nach einem der Ansprüche 1 bis 4, **dadurch gekennzeichnet, dass** der Rezeptor (5a, 5b) ein Biotin und der Ligand (7a, 7b) Streptavidin ist, und dass das Enzym (8) eine Proteinase ist.

6. Verfahren nach einem der Ansprüche 1 bis 5, **dadurch gekennzeichnet, dass** der Rezeptor (5a, 5b) ein Polysaccarid, ein Lipid, ein Digoxygenin und/oder ein Kohlehydrat und der Ligand (7a, 7b) ein Protein, insbesondere ein Antikörper ist, und dass das Enzym (8) eine Proteinase ist.

7. Verfahren nach einem der Ansprüche 1 bis 6, **dadurch gekennzeichnet,**
- **dass** mindestens ein Biosensor (1) bereitgestellt wird, der eine Trägeroberfläche (3) aufweist, auf der wenigstens ein für den Liganden (7a, 7b) bindungsspezifischer Rezeptor (5a, 5b) immobilisiert ist,
- **dass** eine erste Probe, die den Ligand (7a, 7b) in einer bekannten ersten Konzentration enthält, derart mit dem Rezeptor (5a, 5b) in Kontakt gebracht wird, dass der mindestens eine Ligand (7a, 7b) an den Rezeptor (5a, 5b) bindet und mit diesem einen Ligand-Rezeptor-Komplex bildet,
- **dass** mindestens ein von der Bindung des Liganden (7a, 7b) an den Rezeptor (5a, 5b) abhängiger erster Messwert erfasst wird,
- **dass** der Biosensor (1) danach regeneriert wird,
- **dass** dann eine zweite Probe, die den Ligand (7a, 7b) in einer zu bestimmenden zweiten Konzentration enthält, derart mit dem Rezeptor (5a, 5b) in Kontakt gebracht wird, dass der mindestens eine Ligand (7a, 7b) an den Rezeptor (5a, 5b) binden und mit diesem einen Ligand-Rezeptor-Komplex bilden kann,
- **dass** mindestens ein von der Bindung des Liganden (7a, 7b) an den Rezeptor (5a, 5b) abhängiger zweiter Messwert erfasst wird,
- und **dass** mit Hilfe der Messwerte und der bekannten ersten Konzentration die zu bestimmende zweite Konzentration ermittelt wird.

## Geänderte Patentansprüche

### Geänderte Patentansprüche gemäss Regel 137(2) EPÜ.

**1.** Verfahren zum Regenerieren eines Biosensors (1), wobei ein Biosensor (1) bereitgestellt wird, der eine Trägeroberfläche (3) aufweist, auf der wenigstens ein Rezeptor (5a, 5b) immobilisiert ist, an den mindestens ein für den Rezeptor (5a, 5b) bindungsspezifischer Ligand (7a, 7b), nämlich ein Protein gebunden ist, der mit dem Rezeptor (5a, 5b) einen Ligand-Rezeptor-Komplex bildet, und wobei der Biosensor (1) regeneriert wird, indem der Ligand (7a, 7b) vom Rezeptor (5a, 5b) getrennt wird, wobei der Ligand-Rezeptor-Komplex zum Regenerieren mit einem Proteinase-Enzym (8) in Kontakt gebracht wird, und wobei das Enzym (8) derart gewählt wird, dass es den Liganden (7a, 7b) in Fragmente (9) katalysiert und gegenüber dem Rezeptor (5a, 5b) inert ist, **dadurch gekennzeichnet, dass** der Rezeptor (5a, 5b) ein Polysaccarid ist.

**2.** Verfahren nach Anspruch 1, **dadurch gekennzeichnet, dass** der Ligand ein Antikörper ist.
